# EUROPÄISCHE PATENTSCHRIFT

(11) **EP 0 827 384 B1**
(45) Veröffentlichungstag und Bekanntmachung des Hinweises auf die Patenterteilung: **21.08.2002**
(21) Anmeldenummer: 96914944.2
(22) Anmeldetag: 18.04.1996
(51) Int. Cl.: A61B 5/022, A61B 3/16, A61B 3/125

(54) **OPHTHALMODYNAMOMETER**
OPHTHALMODYNAMOMETER
OPHTALMODYNAMOMETRE

(30) Priorität: 21.04.1995 DE 19514796
(43) Veröffentlichungstag der Anmeldung: 11.03.1998
(73) Patentinhaber: Löw, Bernhard, Dr. med., 66333 Völklingen (DE)
(72) Erfinder: Löw, Bernhard, Dr. med., 66333 Völklingen (DE)
(74) Vertreter: Hering, Hartmut, Dipl.-Ing.
(86) Internationale Anmeldenummer: EP9601627
(87) Internationale Veröffentlichungsnummer: WO96032884

(56) Entgegenhaltungen:
- EP-A- 0 114 499
- EP-A- 0 327 693
- DE-A- 4 235 079
- DE-B- 1 055 175
- FR-A- 1 035 662

## Beschreibung

Die Erfindung bezieht sich auf ein Ophthalmodynamometer.

Mit Ophthalmodynamometrie wird in der Medizin die Blutdruckmessung am Auge bezeichnet. Die Blutdruckmessung erfolgt auf unblutige Weise. Gemäß dem hierzu allgemein bekannten Prinzip wird der extravasale Gewebsdruck kontinuierlich so lange erhöht, bis infolge einer Entspannung der Gefäßwand bei Überschreiten des diastolischen oder minimalen Blutdrucks das Blutgefäß intermittierend kollabiert und bei Überschreiten des systolischen oder maximalen Blutdrucks das Blutgefäß dauernd verschlossen bleibt. Die Ophthalmodynamometrie ist sowohl zeit- als auch personalintensiv, da hierfür bisher mindestens zwei Fachkräfte erforderlich waren, von denen eine das Instrumentarium zur Veränderung des intraokularen Drucks bediente, und die andere die Gefäßpulsationen am Augenhintergrund (Fundus) durch indirekte Ophthalmoskopie beobachtete.

Aus FR 1 035 662 oder EP 0 327 693 A1 sind Einrichtungen für die Durchführung der Ophthalmodynamometrie bekannt, welche Mittel zur Druckerzeugung, wie einen Andrückkörper zur Druckbeaufschlagung eines zu untersuchenden Auges und zur Steigerung des Augeninnendrucks sowie Einrichtungen zur Druckerfassung hierbei umfassen. Bei beiden Druckschriften wird ein gesondert und speziell ausgebildeter Andrückkörper eingesetzt. Dieser Andrückkörper ist annähernd zylinderförmig ausgebildet, durch welchen hindurch mit Hilfe einer Spaltlampe, einem Kondensorsystem und einem teildurchlässigen Spiegel eine Beleuchtung erfolgt und zur Untersuchung eine Betrachtungsoptik zugeordnet ist. Ein solches System ist apparativ sehr aufwendig und bedarf einer großen Erfahrung beim Einsatz zur Augenuntersuchung. Ferner ist der Andrückkörper derart gestaltet, daß die Lichtreflektion an der Oberfläche der Hornhaut weitgehend eliminiert werden. Ferner ist eine Untersuchung des Augenhintergrunds nur in den rotationssymmetrisch konkaven Kontaktbereichen des Andrückkörpers mit der Hornhaut des zu untersuchenden Auges möglich, so daß nur der zentrale Bereich des Augenhintergrunds eingesehen werden kann, während die Netzhautperipherie bei der Untersuchung nicht erfaßt werden kann. Die vom Auge abgewandte Fläche des Andrückkörpers ist eben und mit einer Antireflektionsschicht versehen. Ferner ist diese Fläche an die optischen Anforderungen für Beleuchtung und Betrachtung des Augenhintergrunds angepaßt. Die bei der Ophthalmodynamometrie über den Andrückkörper aufgebrachte Druckkraft wird über eine mit einer Membran verschlossene Öffnung auf der Mitte der Hornhaut über eine inkompressible Flüssigkeit als Drucksensor erfaßt. Somit hat der Andrückkörper auch eine dahingehend spezielle Ausgestaltung, daß er einerseits eine Membrane hat und andererseits einen hermetisch dicht abgeschlossenen Raum besitzt, welcher mit einer inkompressiblen Flüssigkeit gefüllt ist. Somit ist auch die Druckerfassungseinrichtung konstruktiv und apparatetechnisch sehr aufwendig ausgelegt.

In DE 42 35 079 ist eine Vorrichtung zum Untersuchen des Auges, insbesondere des menschlichen Auges beschrieben, welche eine MeB-Sonde aufweist, welche in oder an einem auf das Auge aufsetzbaren Halter angeordnet ist. Mit Hilfe einer Ultraschall-Dopplersonde oder einer Laser-Sonde sollen Funktionen und Zustände des Auges zuverlässig und reproduzierbar erfaßt und gemessen werden.

Hierbei soll insbesondere die Durchblutung des menschlichen Auges erfaßt werden, während eine Untersuchung des Augenhintergrunds und eine Druckbeaufschlagung des Auges nicht angesprochen sind.

Demgegenüber liegt der vorliegenden Erfindung die Aufgabe zugrunde, ein Ophthalmodynamometer bereitzustellen, welches mit möglichst geringem apparatetechnischem, konstruktiven Aufwand eine zuverlässige Durchführung der Ophthalmodynamometrie gestattet.

Nach der Erfindung wird hierzu ein Ophthalmodynamometer mit einem Andrückkörper zur Druckbeaufschlagung eines zu untersuchenden Auges und zur Steigerung des Augeninnendrucks bereitgestellt, welcher sich durch die Merkmale des Kennzeichens des Patentanspruchs 1 auszeichnet.

Bei dem erfindungsgemäßen Ophthalmodynamometer wird ein an sich in der Augenmedizin bekanntes Funduskontaktglas eingesetzt, welches zugleich zur Druckbeaufschlagung und als Andrückkörper zur Steigerung des Augeninnendrucks als auch zur Untersuchung des gesamten Augenhintergrunds dient, wobei die Erfassungseinrichtung für die auf das Funduskontaktglas aufgebrachte Druckkraft in Wirkverbindung mit dem vom Auge abgewandten Ende des Funduskontaktglases steht. Bei dem erfindungsgemäßen Ophthalmodynamometer sind daher in das Funduskontaktglas die Funktion zur Beobachtung und zur Untersuchung des gesamten Augenhintergrunds einschließlich der Netzhautperipherie als auch die Funktion eines Andrückkörpers vereint, so daß man weder eine gesonderte Betrachtungsoptik noch eine Beleuchtung und auch keinen gesondert gestalteten Andrückkörper benötigt. Da ferner die Erfassungseinrichtung für die auf das Funduskontaktglas aufgebrachte Druckkraft in Wirkverbindung mit dem vom Auge abgewandten Ende des Funduskontaktglases steht, beeinträchtigt bei dem erfindungsgemäßen Ophthalmodynamometer diese Erfassungseinrichtung nicht die Untersuchung des Augenhintergrunds mit Hilfe des Funduskontaktglases.

Somit wird mit Hilfe eines kompakt und einfach ausgelegten Ophthalmodynamometers auf apparatetechnische äußerst einfache Weise eine zuverlässig Durchführung der Ophthalmodynamometrie ermöglicht.

Da bei dem Ophthalmodynamometer nach der Erfindung der Druck auf das Auge über das Funduskontaktglas in die darunter liegende Hornhaut aufgebracht wird, sind die Gefahren von Netzhautbeschädigungen bei der Druckkraftbeaufschlagung im Vergleich zu einem Andrückkörper äußerst gering, mit dem die Lederhaut mit der darunter liegenden Netzhaut durch den Stempeldruck des Andrückkörpers gedehnt und abgeplattet wird. Somit läßt sich das Ophthalmodynamometer nach der Erfindung auch mit beträchtlich reduziertem Verletzungsrisiko betreiben.

Gemäß bevorzugten Ausführungsformen ist die Erfassungseinrichtung am vom Auge abgewandten Ende des Funduskontaktglases angeordnet, oder sie ist im vom Auge abgewandten Ende des Funduskontaktglases integriert. Somit kann die auf das Auge aufgebrachte Druckkraft unmittelbar am Funduskontaktglas erfaßt und abgelesen werden. Hierbei kann die vorhandene Auslegung des Funduskontaktglases in dem Bereich unverändert belassen werden, an welchem das Funduskontaktglas auf das zu untersuchende Auge aufgesetzt wird.

Weitere vorteilhafte Ausgestaltungen der Erfindung sind in den Ansprüchen 4 bis 6 wiedergegeben.

Nach der Erfindung ist es wesentlich, daß die Druckkraftbeaufschlagung mit ein und demselben Mittel, wie mit dem Funduskontaktglas erfolgt, welches auch zur Augenhintergrundsbeobachtung eingesetzt wird, und daß die auf das Auge mit Hilfe des Funduskontaktglases aufgebrachte Druckkraft zugleich erfaßt wird, um die am Augenhintergrund mit Hilfe des Funduskontaktglases beobachteten Gefäßpulsationen den Beaufschlagungsdruckwerten zuordnen zu können, wobei die auf das Auge ausgeübte Druckkraft eine direkte proportionale Steigerung des Augeninnendrucks mit Hilfe des auf das Auge aufgesetzten Funduskontaktglases gestattet. Somit erhält man ein unproblematisch verwirklichbares Betriebsverfahren für ein Ophthalmodynamometer, welches ferner noch eine kompakt ausgelegte Einheit bildet.

Die Erfindung wird nachstehend an Hand von bevorzugten Ausführungsformen unter Bezugnahme auf die beigefügte Zeichnung näher erläutert. Darin zeigt:
- Fig. 1: eine schematische Ansicht einer ersten Ausführungsform eines Ophthalmodynamometers,
- Fig. 2: eine schematische Ansicht einer zweiten Ausführungsform eines Ophthalmodynamometers, und
- Fig. 3: eine schematische Ansicht einer dritten Ausführungsform eines Ophtalmodynamometers.

An Hand diesen Figuren der Zeichnung wird das Ophthalmodynamometer erörtert. In den Figuren der Zeichnung sind gleiche oder ähnliche Teile mit denselben Bezugszeichen versehen.

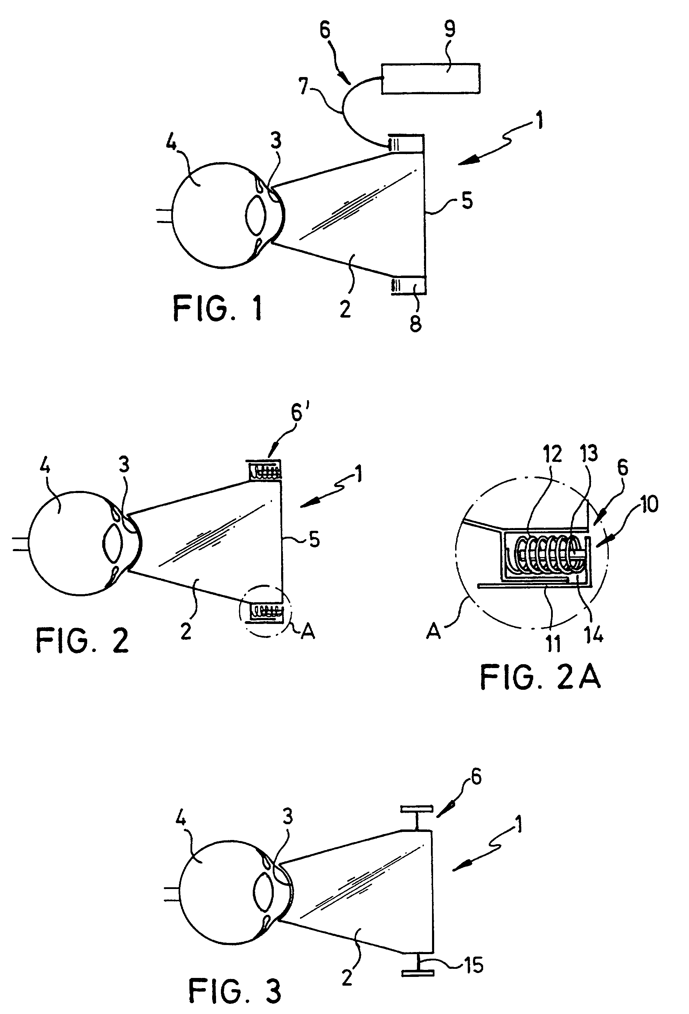

In Figur 1 ist ein Ophthalmodynamometer insgesamt mit 1 bezeichnet. Dieses umfaßt ein in der Seitenansicht schematisch dargestelltes Funduskontaktglas 2, welches an einem vorderen Ende 3 bzw. einem dem Auge 4 zugewandten Ende 3 in geeigneter Weise so geformt ist, daß es auf das zu untersuchende Auge 4 so aufsetzbar ist, daß keine Zilien eingeklemmt werden. Das Funduskontaktglas 2 ist wie dargestellt trichterförmig ausgebildet. Am vom Auge 4 abgewandten Ende 5 des Funduskontaktglases 2 wird dieses von einem nicht näher dargestellten Untersucher gehandhabt. Der Untersucher geht zum Betrieben des Ophthalmodynamometers derart vor, daß das Funduskontaktglas 2 auf das Auge 4 aufgesetzt wird. Zur Steigerung des Augeninnendrucks wird auf das Auge 4 eine Druckkraft mittels des Funduskontaktglases 2 dadurch ausgeübt, daß der Untersucher auf das Ende 5 des Funduskontaktglases 2 drückt. Die mit Hilfe des Funduskontaktglases 2 aufgebrachte Druckkraft ist direkt proportional zur Steigerung des hierdurch im Auge 4 erzeugten Augeninnendrucks. Bei der nach Figur 1 gezeigten Ausführungsform ist eine insgesamt mit 6 bezeichnete Erfassungseinrichtung für die auf das Funduskontaktglas 2 aufgebrachte Druckkraft vorgesehen, welche mit dem Ende 5 des Funduskontaktglases 2 in Wirkverbindung steht. Bei der Ausführungsform nach Figur 1 erfaßt die Erfassungseinrichtung 6, wie ein Membranmanometer, welches über eine schematisch dargestellte Verbindungsleitung 7 an einen Raum 8 angeschlossen ist, welcher das Funduskontaktglas 2 am Ende 5 außenseitig umgibt, diese aufgebrachte Druckkraft. Das Membranmanometer selbst, welches mit 9 bezeichnet ist, ist nur schematisch dargestellt.

Auf diese Weise wird die auf das Funduskontaktglas 2 vom Untersucher aufgebrachte Druckkraft mittels des Funduskontaktglases 2 erfaßt, und zugleich gestattet das Funduskontaktglas 2 eine Beobachtung beispielsweise der Gefäßpulsationen am Augenhintergrund (Fundus). Somit lassen sich auf einfache Weise die Phänomene der Gefäße am Augenhintergrund beobachten und erkennen, welche für das Erreichen des minimalen und des maximalen Blutdruckes typisch sind, und zwar mit Hilfe des Funduskontaktglases 2. Die Steigerung des Augeninnendrucks wird ebenfalls mit Hilfe des Funduskontaktglases 2 erzielt. Somit läßt sich beim Betreiben des Ophthalmodynamometers 1 auf vereinfachte Weise beispielsweise von nur einem Untersucher zuverlässig eine Ophthalmodynamometrie vornehmen. Das Ophthalmodynamometer 1 läßt sich unkompliziert handhaben und ist einfach ausgelegt, indem es im wesentlichen ein Funduskontaktglas 2 umfaßt, welches einerseits zur Augenhintergrundsbeobachtung und andererseits zur Druckkraftbeaufschlagung des Auges 4 genutzt wird. Zugleich wird am Funduskontaktglas 2 die auf dasselbe vom Untersucher ausgeübte Druckkraft mit Hilfe der zugeordneten Erfassungseinrichtung 6 gemessen und erfaßt.

Bei der in Figur 2 schematisch dargestellten Ausführungsform wird in Abweichung zu der Ausführungsform nach Figur 1 als Erfassungseinrichtung 6' eine induktive Wegmeßeinrichtung dargestellt. In Figur 2a ist ein Beispiel einer derartigen induktiven Wegmeßeinrichtung in vergrößerter Ansicht gezeigt, welche dort insgesamt mit 10 bezeichnet ist. Am mit 5 bezeichneten Ende des Funduskontaktglases 2 ist ein relativ bewegliches Teil 11 gezeigt, welches über eine Feder 12 am Funduskontaktglas 2 abgestützt ist. Ferner sind Induktionsspulen 13 in dem zwischen dem Funduskontaktglas 2 und dem relativ beweglichen Teil 11 gebildeten Raum 14 vorgesehen. Mit Hilfe dieser induktiven Wegmeßeinrichtung 10 läßt sich dann die auf das Funduskontaktglas 2 ausgeübte Druckkraft messen und erfassen.

Bei der in Figur 3 schematisch dargestellten Ausführungsform ist eine Erfassungseinrichtung 6" für die auf das Funduskontaktglas 2 ausgeübte Druckkraft vorgesehen, welche Dehnungsmeßstreifen 15 umfaßt, welche am Ende 5 des Funduskontaktglases 2 vorgesehen sind.

Natürlich handelt es sich bei den vorstehend an Hand den Figuren 1 bis 3 gezeigten und erläuterten Erfassungseinrichtungen 6, 6' und 6" nur um bevorzugte Ausführungsformen und Beispiele, und der Fachmann kann selbtverständlich ohne weiteres geeignete Abänderungen vornehmen und zur Erfassung der auf das Funduskontaktglas 2 aufgebrachten Druckkraft entsprechend geeignete Einrichtungen wählen, welche sich in platzsparender Weise am Funduskontaktglas 2 vorsehen oder in dasselbe integrieren lassen. Wesentlich hierbei ist es, daß das Funduskontaktglas 2 zur Druckkraftbeaufschlagung einerseits genutzt wird, und zugleich auch eine Erfassung der Größe der aufgebrachten Druckkraft auf einfach und zuverlässige Weise gestattet.

Wesentlich bei dem Ophthalmodynamometer ist es, daß das Funduskontaktglas 2 für die Beobachtung des Augenhintergrunds des Auges 4 und zugleich dazu genutzt wird, auf das Auge 4 eine Druckkraft zur Steigerung des Augeninnendrucks aufzubringen. Ferner dient das Funduskontaktglas 2 wenigstens als Träger für eine Erfassungseinrichtung 6, 6', 6" für die auf das Funduskontaktglas 2 aufgebrachte Druckkraft, wobei zur möglichst genauen Erfassung der Druckkraft eine solche Erfassung und Messung erfolgen sollte, die direkt in unmittelbarem Wirkzusammenhang mit dem Funduskontaktglas 2 bei der Druckkraftbeaufschlagung steht.

### Bezugszeichenliste

- 1: Vorrichtung für Ophthalmodynamometrie insgesamt
- 2: Kontaktglas
- 3: vorderes Ende des Kontaktglases 2
- 4: Auge
- 5: vom Auge 4 abgewandtes Ende des Kontaktglases 2
- 6, 6', 6": Erfassungseinrichtung insgesamt
- 7: Verbindungsleitung
- 8: Raum
- 9: Membranmanometer
- 10: Induktive Wegmeßeinrichtung insgesamt
- 11: Relativ bewegliches Teil
- 12: Feder
- 13: Induktionsspulen
- 14: Raum
- 15: Dehnungsmeßstreifen

## Patentansprüche

1. Ophthalmodynamometer mit einem Andrückkörper zur Druckbeaufschlagung eines zu untersuchenden Auges und zur Steigerung des Augeninnendrucks, **dadurch gekennzeichnet, daß** der Andrückkörper von einem auf das Auge (4) aufsetzbaren und zur Beobachtung des gesamten Augenhintergrunds dienenden Funduskontaktglas (2) gebildet wird, und daß mit dem vom Auge (4) abgewandten Ende (5) des Funduskontaktglases (2) eine Erfassungseinrichtung (6, 6', 6") für die auf das Funduskontaktglas (2) aufgebrachte Druckkraft in Wirkverbindung steht.

2. Ophthalmodynamometer nach Anspruch 1, **dadurch gekennzeichnet, daß** die Erfassungseinrichtung (6, 6', 6") am vom Auge (4) abgewandten Ende (5) des Funduskontaktglases (2) angeordnet ist.

3. Ophthalmodynamometer nach Anspruch 1, **dadurch gekennzeichnet, daß** die Erfassungseinrichtung (6, 6', 6") im vom Auge (4) abgewandten Ende (5) des Funduskontaktglases (2) integriert ist.

4. Ophthalmodynamometer nach einem der Ansprüche 1 bis 3, **dadurch gekennzeichnet, daß** die Erfassungseinrichtung (6) ein Membranmanometer (9) umfaßt.

5. Ophthalmodynamometer nach einem der Ansprüche 1 bis 3, **dadurch gekennzeichnet, daß** die Erfassungseinrichtung (6') eine induktive Wegmessung (10) umfaßt.

6. Ophthalmodynamometer nach einem der Ansprüche 1 bis 3, **dadurch gekennzeichnet, daß** die Erfassungseinrichtung (6") Dehnungsmeßstreifen (15) umfaßt.

## Claims

1. Ophthalmodynamometer having a contact body for applying pressure to an eye to be examined and for increasing the intra-ocular pressure, **characterised in that** the contact body is formed by a fundus contact glass (2) which can be placed on the eye (4) and which serves to observe the entire ocular fundus, and **in that** a recording device (6, 6', 6") for the compressive force applied to the fundus contact glass (2) cooperates with the end (5) of the fundus contact glass (2) facing away from the eye (4).

2. Ophthalmodynamometer according to claim 1, **characterised in that** the recording device (6, 6', 6") is arranged at the end (5) of the fundus contact glass (2) facing away from the eye (4).

3. Ophthalmodynamometer according to claim 1, **characterised in that** the recording device (6, 6', 6") is integrated in the end (5) of the fundus contact glass (2) facing away from the eye (4).

4. Ophthalmodynamometer according to one of claims 1 to 3, **characterised in that** the recording device (6) comprises a membrane manometer (9).

5. Ophthalmodynamometer according to one of claims 1 to 3, **characterised in that** the recording device (6') comprises an inductive position sensor (10).

6. Ophthalmodynamometer according to one of claims 1 to 3, **characterised in that** the recording device (6") comprises wire strain gauges (15).

## Revendications

1. Sphygmomanomètre oculaire muni d'un corps de pression destiné à imprimer une pression à un oeil en cours d'examen et à augmenter la pression oculaire interne, **caractérisé en ce que** le corps de pression est construit à partir d'un verre de contact scléral (2) apte à être posé sur l'oeil (4) et servant à l'observation de l'ensemble du fond de l'oeil, et **en ce qu'**un dispositif de contrôle (6, 6', 6") de la force de pression appliquée au verre de contact scléral (2) est en liaison avec l'extrémité (5) du verre de contact scléral (2) qui est la plus éloignée de l'oeil (4).

2. Sphygmomanomètre oculaire selon la revendication 1, **caractérisé en ce que** le dispositif de contrôle (6, 6', 6") est disposé à l'extrémité (5) du verre de contact scléral (2) qui est la plus éloignée de l'oeil (4).

3. Sphygmomanomètre oculaire selon la revendication 1, **caractérisé en ce que** le dispositif de contrôle (6, 6', 6") est intégré dans l'extrémité (5) du verre de contact scléral (2) qui est la plus éloignée de l'oeil (4).

4. Sphygmomanomètre oculaire selon l'une des revendications 1 à 3, **caractérisé en ce que** le dispositif de contrôle (6) comprend un manomètre à membrane (9).

5. Sphygmomanomètre oculaire selon l'une des revendications 1 à 3, **caractérisé en ce que** le dispositif de contrôle (6') comprend un capteur à induction (10) pour mesurer une amplitude de déplacement.

6. Sphygmomanomètre oculaire selon l'une des revendications 1 à 3, **caractérisé en ce que** le dispositif de contrôle (6") comprend des extensomètres à bande (15).
